# EUROPÄISCHE PATENTSCHRIFT

(11) **EP 1 110 970 B1**
(45) Veröffentlichungstag und Bekanntmachung des Hinweises auf die Patenterteilung: **14.05.2008**
(21) Anmeldenummer: 00125846.6
(22) Anmeldetag: 25.11.2000
(51) Int. Cl.: C07K 16/26, G01N 33/74, A61K 39/395, C12N 5/12, C07K 14/585

(54) **Gegen Procalcitonin gerichtete Antikörper, ihre Herstellung und Verwendung**
Anti-Procalcitonin antibodies, their production and use
Anticorps anti-procalcitonin, procédé de production et utilisation

(30) Priorität: 22.12.1999 DE 19962417; 03.04.2000 DE 10016277; 22.08.2000 DE 10041215
(43) Veröffentlichungstag der Anmeldung: 27.06.2001
(73) Patentinhaber: Dade Behring Marburg GmbH, 35001 Marburg (DE)
(72) Erfinder: Althaus, Harald, 35083 Wetter (DE); Götz, Walter, Dr., 35117 Münchhausen (DE)

(56) Entgegenhaltungen:
- WO-A-94/04927
- WO-A-98/33524
- GHILLANI P. ET AL: "Monoclonal antipeptide antibodies as tools to dissect closely related gene products: A model using peptides encoded by the calcitonin gene." JOURNAL OF IMMUNOLOGY, (1988) 141/9 (3156-3163). , XP002161903

## Beschreibung

Die Erfindung betrifft gegen Procalcitonin gerichtete Antikörper, ihre Herstellung und Verwendung.

Procalcitonin ("pCT") ist ein aus 116 Aminosäuren bestehendes Protein mit einem Molekulargewicht von ca. 13000 Dalton. Es stellt das Prohormon von Calcitonin dar, welches unter normalen Stoffwechselbedingungen in den C-Zellen der Schilddrüse gebildet und sezerniert wird. Die Synthese von pCT und Calcitonin beginnt mit der Translation eines 141 Aminosäuren umfassenden Vorläuferpeptides, dem Preprocalcitonin ("Pre-pCT"). Die Aminosäuresequenz von menschlichem Pre-pCT wurde 1984 von Moullec et al. in FEBS Letters, 167:93-97 beschrieben. Nach Abspaltung des Signalpeptides (ersten 25 Aminosäuren von Pre-pCT) entsteht pCT. Bei Gesunden wird durch spezifische Proteolyse aus pCT intrazellulär das Hormon Calcitonin (Aminosäuren 60-91 der pCT-Aminosäuresequenz) sowie das N-Procalcitonin (Aminosäuren 1-57 der pCT-Aminosäuresequenz) und das Katacalcin (Aminosäuren 96-116 der pCT-Aminosäuresequenz) gebildet (s.a. Conlon et al. (1988) Biochem. J., 256:245-250). Das pCT sowie dessen Fragmente wurden insbesondere bei bestimmten Tumorerkrankungen (Ghillani et al. (1989) Cancer Research, 49:6845-6851) sowie bei Sepsis (EP-B1-0 656 121) und SIRS ("systemic inflammatory response syndrome") (Snider et al. (1997) J. Investig. Med., 45:552-560) in erhöhter Konzentration im Serum bzw. Plasma von Patienten nachgewiesen.

Bei der typischen Sepsis werden Bakterien von einem Herd aus ständig oder schubweise in die Blutbahn abgegeben. Die klinischen Erscheinungen werden durch Endotoxin oder durch andere pyrogene und toxische Substanzen in ihrer Interaktion mit Körpermechanismen verursacht. Der akute Einbruch löst Schüttelfrost und in schweren Fällen eine Schockreaktion aus. Sonderformen des septischen Schocks sind das Waterhouse-Friderichsen-Syndrom und das Toxinschocksyndrom (TSS).

Das TSS ist als ein akutes Krankheitsbild bei Staphylokokken-Infektionen bekannt, das durch ein spezielles Staphylokokken-Toxin hervorgerufen wird. Eine schwere Sepsis entwickelt sich relativ häufig bei Patienten mit schweren Grunderkrankungen wie z.B. Tumorerkrankungen, schweren Verbrennungen und Traumen.

Die Bedeutung des Nachweises der Erreger im Blut ("positive Blutkultur, Bakteriämie") ist für die Sepsisdiagnose in den Hintergrund getreten, da in der Regel nur in 20 bis 40% der Sepsisfälle die Blutkultur positiv ist. Der Sepsisbegriff hat sich deshalb einem Wandel unterzogen. Der moderne Begriff "Sepsis" beschreibt ein klinisches Syndrom, welches in der Regel Fieber, Leukozytose, Bewußtseinsveränderungen, einen hyperdynamischen Kreislauf ("warmer Schock") und einen hypermetabolischen Zustand umfaßt, wobei eine positive Blutkultur als Voraussetzung für die Diagnose einer Sepsis nicht mehr erforderlich ist.

In WO 98/33524 wird vorgeschlagen, Antikörper, die an pCT binden, zur Therapie von Sepsis und SIRS einzusetzen.

Über lange Jahre wurden polyklonale Antikörper, die durch Immunisierung mit Calcitonin gewonnen wurden, zum Nachweis des sogenannten immunreaktiven Calcitonins, welches neben Calcitonin auch das Procalcitonin und weitere Bruchstücke des Procalcitonins umfaßt, verwendet. Durch die Immunisierung mit synthetischen Peptiden, deren Aminosäuresequenzen mit den Sequenzen von Teilabschnitten des Procalcitonins übereinstimmt, gelang es verschiedene monoklonale Antikörper herzustellen, die an verschiedene Epitope des Calcitonins und Katacalcins binden (Ghillani et al. (1988) J. Immunol., 141:3156-3163).

Auf Basis dieser Antikörper wurden auch Sandwich-lmmunoassays zum Nachweis von pCT bzw. Calcitonin in Serumproben entwickelt. Zum Nachweis von Calcitonin-Vorläufermolekülen wurde eine Kombination eines Anti-Katacalcin-Antikörpers mit einem Anti-Calcitonin-Antikörper vorgeschlagen (EP-B1-0 656 121). Eine solche Methode hat jedoch den Nachteil, daß zum pCT-Nachweis mindestens zwei Antikörper, die an möglichst weit auseinanderliegende pCT-Bereiche binden müssen, benötigt werden.

Für den Fachmann stellt sich daher die Aufgabe, andere spezifische Bindungspartner bereitzustellen, die es erlauben, pCT auch nur mit einem spezifischen Bindungspartner nachweisen zu können.

Diese Aufgabe wird durch die Bereitstellung der erfindungsgemäßen Antikörper gelöst, die an Procalcitonin nicht jedoch an freies Calcitonin, freies Katacalcin und freies N-Procalcitonin binden. Bevorzugt sind im Sinne der Erfindung Antikörper, die an ein Peptid mit der Aminosäuresequenz Val-Gly-Ala-Pro-Gly-Lys-Lys-Arg-Asp-Met-Ser-Ser binden.

Im Gegensatz zu den bisher bekannten Antikörpern, die pCT und zugleich aber auch freies Calcitonin oder freies Katacalcin oder freies N-Procalcitonin binden, können die erfindungsgemäßen Antikörper auch alleine in kompetitiven Testverfahren oder in immunhistochemischen Methoden zum spezifischen pCT-Nachweis eingesetzt werden. Hinzu kommt, daß die erfindungsgemäßen Antikörper zur affinitätschromatographischen Aufreinigung von pCT aus einer Probe, die auch pCT-Spaltprodukte enthält, besonders geeignet sind.

Obwohl Ghillani et al. bei ihren Immunisierungsversuchen auch Peptide, die die Aminosäuresequenz Pro-Gly-Lys-Lys-Arg-Asp bzw. die Aminosäuresequenz Val-Gly-Ala-Pro-Gly-Lys-Lys-Arg-Asp-Met-Ser-Ser enthalten, eingesetzt haben, gelang ihnen trotz intensiven Bemühens um die Identifizierung der pCT-Epitope nur die Bereitstellung von Antikörpern, die entweder das "reife" Calcitonin oder pCT gemeinsam mit Calcitonin bzw. pCT gemeinsam mit Katacalcin erkennen.

Überraschenderweise ist es nun gelungen, Antikörper zu erzeugen, die an Procalcitonin nicht jedoch an freies Calcitonin, freies Katacalcin und freies N-Procalcitonin binden.

Im folgenden werden spezifische Ausführungsformen der Erfindung näher erläutert:

Gegenstand der Erfindung sind solche Antikörper, die dadurch gekennzeichnet sind, daß diese Antikörper Procalcitonin nicht jedoch freies Calcitonin, freies Katacalcin und freies N-Procalcitonin binden.

Unter dem Begriff "Antikörper" ist generell ein Immunglobulin, z.B. ein Immunglobulin der Klasse bzw. Subklasse IgA, IgD, IgE, IgG₁, IgG₂ₐ, IgG_{2b}, IgG₃, IgG₄, lgM, zu verstehen. Ein Antikörper weist mindestens eine Bindungsstelle (häufig Paratop genannt) für ein Epitop (häufig auch antigene Determinante genannt) auf einem Antigen oder Hapten auf. Ein solches Epitop ist z.B. durch seine räumliche Struktur und/oder durch das Vorhandensein von polaren und/oder apolaren Gruppen gekennzeichnet. Die Bindungsstelle des Antikörpers ist komplementär zum Epitop. Die Antigen-Antikörper-Reaktion bzw. die Hapten-Antikörper-Reaktion funktioniert nach dem sogenannten "Schlüssel-Schlüsselloch-Prinzip", und ist in der Regel in einem hohen Grad spezifisch, d.h. die Antikörper vermögen kleine Abweichungen in der Primärstruktur, in der Ladung, in der räumlichen Konfiguration und der sterischen Anordnung des Antigens oder Haptens zu unterscheiden. Insbesondere die sogenannten "complementarity determining regions" des Antikörpers tragen zur Bindung des Antikörpers an das Antigen oder Hapten bei.

Der Begriff "Antigene" umfaßt monovalente und polyvalente Antigene. Ein polyvalentes Antigen ist ein Molekül oder ein Molekülkomplex, an das/den mehr als ein Immunglobulin gleichzeitig binden kann, während bei einem monovalenten Antigen jeweils nur ein einziger Antikörper zur selben Zeit binden kann. Als Hapten wird üblicherweise ein Molekül bezeichnet, das nicht für sich allein immunogen ist, sondern das zu Immunisierungszwecken üblicherweise an einen Träger gebunden wird.

Unter dem Begriff Antikörper sind im Sinne dieser Erfindung nicht nur komplette Antikörper zu verstehen sondern ausdrücklich auch Antikörperfragmente, wie z.B. Fab, Fv, F(ab')₂, Fab'; sowie auch chimäre, humanisierte, bi- oder oligospezifische, oder "single chain" Antikörper; des weiteren auch Aggregate, Polymere und Konjugate von Immunglobulinen und/oder deren Fragmenten, sofern die Bindungseigenschaften an das Antigen oder Hapten erhalten sind. Antikörperfragmente lassen sich beispielsweise durch enzymatische Spaltung von Antikörpern mit Enzymen wie Pepsin oder Papain herstellen. Antikörperaggregate, - polymere und -konjugate können durch vielfältige Methoden generiert werden, z.B. durch Hitzebehandlung, Umsetzung mit Substanzen wie Glutaraldehyd, Reaktion mit immunglobulin-bindenden Molekülen, Biotinylierung von Antikörpern und anschließende Reaktion mit Streptavidin oder Avidin, etc..

Bei den Antikörpern im Sinne dieser Erfindung handelt es sich um die pCTbindenden Antikörper, die von der Hybridomazelllinie 98-31/04 produziert werden. Diese Hybridomazelllinie wurde am 16.12.1999 bei der DSMZ-Deutsche Sammlung von Mikroorganismen und Zellkulturen GmbH, Mascheroder Weg 1b, Braunschweig, Deutschland, unter der Eingangsnummer DSM ACC2437 hinterlegt.

Gegenstand dieser Erfindung ist auch ein erfindungsgemäßer Antikörper, der mit einer Festphase und/oder einer Komponente eines signalbildenden Systems assoziiert ist.

Der Begriff "Festphase" im Sinne dieser Erfindung beinhaltet einen Gegenstand, der aus porösem und/oder nicht porösem, in der Regel wasserunlöslichem Material besteht und die unterschiedlichsten Formen aufweisen kann, wie z,B. Gefäß, Röhrchen, Mikrotitrationsplatte, Kugel, Mikropartikel, Stäbchen, Streifen, Filter- oder Chromatographiepapier, etc. In der Regel ist die Oberfläche der Festphase hydrophil oder kann hydrophil gemacht werden. Die Festphase kann aus den unterschiedlichsten Materialien bestehen wie z.B. aus anorganischen und/oder aus organischen Materialien, aus synthetischen, aus natürlich vorkommenden und/oder aus modifizierten natürlich vorkommenden Materialien. Beispiele für Festphasenmaterialien sind Polymere wie z.B. Zellulose, Nitrozellulose, Zelluloseacetat, Polyvinylchlorid, Polyacrylamid, vernetzte Dextranmoleküle, Agarose, Polystyrol, Polyethylen, Polypropylen, Polymethacrylat oder Nylon; Keramik; Glass; Metalle, insbesondere Edelmetalle wie Gold und Silber; Magnetit; Mischungen oder Kombinationen derselben; etc. Auch Zellen, Liposomen oder Phospholipidvesikel, sind vom Begriff Festphase miterfaßt.

Die Festphase kann einen Überzug aus einer oder mehreren Schichten aufweisen, z.B. aus Proteinen, Kohlehydraten, lipophilen Substanzen, Biopolymeren, organischen Polymeren oder Mischungen hiervon, um beispielsweise die unspezifische Bindung von Probenbestandteilen an die Festphase zu unterdrücken oder zu verhindern, oder um beispielsweise Verbesserungen zu erreichen hinsichtlich der Suspensionsstabilität von partikulären Festphasen, der Lagerstabilität, der formgebenden Stabilität oder der Resistenz gegen UV-Licht, Mikroben oder sonstige zerstörend wirkender Agenzien.

Bei einem "signalbildenden System" kann es sich um eine oder mehrere Komponenten handeln, wobei es sich wenigstens bei einer Komponente um ein nachweisbares Label handelt. Als Label ist jedes Molekül zu verstehen, das selbst ein Signal produziert oder die Produktion eines Signals induzieren kann wie z.B. eine fluoreszierende Substanz, eine radioaktive Substanz, ein Enzym, oder eine chemilumineszierende Substanz . Das Signal kann beispielsweise anhand der Enzymaktivität, der Lumineszenz, der Lichtabsorption, der Lichtstreuung, der ausgestrahlten elektromagnetischen oder radioaktiven Strahlung, oder einer chemischen Reaktion nachgewiesen oder gemessen werden.

Ein Label vermag selbst ein nachweisbares Signal zu erzeugen, so daß keine weiteren Komponenten notwendig sind. Viele organische Moleküle absorbieren ultraviolettes und sichtbares Licht, wobei durch die Lichtabsorption übertragene Energie diese Moleküle in einen angeregten Energiezustand kommen können, und die absorbierte Energie in Form von Licht einer anderen Wellenlänge als der des eingestrahlten Lichts abgeben. Wieder andere Label können direkt ein nachweisbares Signal erzeugen wie z.B. radioaktive Isotope oder Farbstoffe.

Wieder andere Label benötigen zur Signalerzeugung weitere Komponenten, d.h. das signalproduzierende System schließt in einem solchen Fall all die für die Signalbildung benötigten Komponenten mit ein wie z.B. Substrate, Coenzyme, Quencher, Beschleuniger, zusätzliche Enzyme, Substanzen die mit Enzymprodukten reagieren, Katalysatoren, Aktivatoren, Cofaktoren, Inhibitoren, lonen etc..

Geeignete Label (s.a. EP-A2-0 515 194; US 5,340,716; US 5,545,834; Bailey et al. (1987) J. Pharmaceutical & Biomedical Analysis 5:649-658) sind beispielsweise Enzyme einschließlich Meerrettichperoxidase, alkalische Phosphatase, Glukose-6-Phosphatdehydrogenase, Alkoholdehydrogenase, Glucoseoxidase, β-Galactosidase, Luciferase, Urease und Acetylcholinesterase; Farbstoffe; fluoreszierende Substanzen einschließlich Fluoresceinisothiocyanat, Rhodamin, Phycoerythrin, Phycocyanin, Ethidiumbromid, 5-Dimethylaminonapthalen-1-sulfonylchlorid und fluoreszierende Chelate von seltenen Erden; chemilumineszierende Substanzen einschließlich Luminol, Isoluminol, Acridiniumverbindungen, Olefin, Enolether, Enamin, Arylvinylether, Dioxen, Arylimidazol, Lucigenin, Luciferin und Aequorin; Sensitizer einschließlich Eosin, 9,10-Dibromoanthracen, Methylen Blau, Porphyrin, Phthalocyanin, Chlorophyll, Rose Bengal; Coenzyme; Enzymsubstrate; radioaktive Isotope einschließlich ¹²⁵I, ¹³¹I, ¹⁴C, ³H, ³²P, ³⁵S, ¹⁴C, ⁵¹Cr, ⁵⁹Fe, ⁵⁷Co und ⁷⁵Se; Partikel einschließlich magnetische Partikel oder Partikel, bevorzugt Latexpartikel, die selbst beispielsweise mit Farbstoffen, Sensitizern, fluoreszierenden Substanzen, chemilumineszierenden Substanzen, Isotopen oder anderen nachweisbaren Labeln markiert sein können; Solpartikel einschließlich Gold- oder Silbersolen; Liposomen oder Zellen, die selbst mit nachweisbaren Labeln markiert sein können; etc.

Ein signalbildendes System kann auch Komponenten umfassen, die bei räumlicher Nähe miteinander in eine nachweisbare Wechselwirkung treten können, z.B. in Form von Energiespendern und Energieempfängern wie beispielsweise Photosensitizer und chemolumineszierende Substanzen (EP-A2-0 515 194), Photosensitizer und Fluorophore (WO 95/06877), radioaktives lod¹²⁵ und Fluorophore (Udenfriend et al. (1985) Proc. Natl. Acad. Sci. 82:8672-8676), Fluorophore und Fluorophore (Mathis (1993) Clin. Chem. 39:1953-1959) oder Fluorophore und Fluoreszenz-Quencher (US 3,996,345).

Unter einer Wechselwirkung zwischen den Komponenten ist die direkte Übertragung von Energie zwischen den Komponenten, z.B. durch Licht- oder Elektronenstrahlung sowie über kurzlebige reaktive chemische Moleküle, miteingeschlossen. Ferner umfaßt sind hiervon auch Vorgänge, bei denen die Aktivität einer Komponente durch eine oder mehrere andere inhibiert oder verstärkt wird, beispielsweise die Hemmung oder Steigerung der Enzymaktivität oder die Hemmung, Steigerung oder Veränderung (z.B. Wellenlängenverschiebung, Polarisation) des von der beeinflußten Komponente ausgesendeten elektromagnetischen Strahlung. Die Wechselwirkung zwischen den Komponenten umfaßt auch Enzymkaskaden. In diesem Fall sind die Komponenten Enzyme, von denen mindestens eines das Substrat für ein anderes liefert, so daß eine maximale oder minimale Reakionsgeschwindigkeit der gekoppelten Substratumsetzung resultiert.

Eine effektive Wechselwirkung zwischen den Komponenten findet in der Regel statt, wenn diese räumlich benachbart vorliegen, also z.B. innerhalb eines Abstandbereiches von wenigen µm, insbesondere innerhalb eines Abstandbereiches von unter 600 nm, bevorzugt unter 400 nm, ganz besonders bevorzugt von unter 200 nm.

Mikropartikel werden häufig als Festphase und/oder als Label genutzt. Unter dem Begriff "Mikropartikel" sind im Sinne dieser Erfindung Teilchen zu verstehen, die einen ungefähren Durchmesser von wenigstens 20 nm und nicht mehr als 20 µm aufweisen, üblicherweise zwischen 40 nm und 10 µm, bevorzugt zwischen 0,1 und 10 µm, besonders bevorzugt zwischen 0,1 und 5 µm, ganz besonders bevorzugt zwischen 0,15 und 2 µm. Die Mikropartikel können regelmäßig oder unregelmäßig geformt sein. Sie können Kugeln, Spheroide, Kugeln mit mehr oder weniger großen Kavitäten oder Poren darstellen. Die Mikropartikel können aus organischem, aus anorganischem Material oder aus einer Mischung oder Kombination von beiden bestehen. Sie können aus einem porösen oder nicht porösen, einem schwellfähigen oder nicht schwellfähigen Material bestehen. Prinzipiell können die Mikropartikel jegliche Dichte haben, bevorzugt sind jedoch Partikel mit einer Dichte, die der Dichte des Wassers nahekommt wie etwa 0,7 bis etwa 1,5 g/ml. Die bevorzugten Mikropartikel sind in wässrigen Lösungen suspendierbar und möglichst lange suspensionsstabil. Sie mögen durchsichtig, teilweise durchsichtig oder undurchsichtig sein. Die Mikropartikel können aus mehreren Schichten bestehen wie beispielsweise die sogenannten "core-and-shell" Partikel mit einem Kern und einer oder mehreren umhüllenden Schichten. Der Begriff Mikropartikel umfaßt beispielsweise Farbstoffkristalle, Metallsolen, Silica-Partikel, Glaspartikel, Magnetpartikel, Polymerteilchen, Öltropfen, Lipidpartikel, Dextran, und Proteinaggregate. Bevorzugte Mikropartikel sind in wässrigen Lösungen suspendierbare und aus wasserunlöslichem Polymermaterial bestehende Partikel, insbesondere aus substituierten Polyethylenen. Ganz besonders bevorzugt sind Latexpartikel z.B. aus Polystyrol, Acrylsäurepolymeren, Methacrylsäurepolymeren, Acrylnitril-Polymeren, Acrylnitril-Butadien-Styrol, Polyvinylacetat-Acrylat, Polyvinylpyridin, Vinylchlorid-Acrylat. Von besonderem Interesse sind Latexpartikel mit reaktiven Gruppen an ihrer Oberfläche wie beispielsweise Carboxyl-, Amino- oder Aldehydgruppen, die eine kovalente Bindung z.B. von spezifischen Bindungspartnern an die Latexpartikel erlauben. Die Herstellung von Latexpartikeln ist beispielsweise in EP 0 080 614, EP 0 227 054 und EP 0 246 446 beschrieben.

Der Begriff "assoziiert" ist breit zu verstehen und umfaßt beispielsweise eine kovalente und eine nicht-kovalente Bindung, eine direkte und eine indirekte Bindung, die Adsorption an eine Oberfläche und den Einschluß in eine Vertiefung oder einen Hohlraum, etc.. Bei einer kovalenten Bindung sind die Antikörper oder Bindungspartner über eine chemische Bindung an die Festphase oder an das Label gebunden. Beispiele für eine nicht-kovalente Bindung sind die Oberflächenadsorption, der Einschluß in Hohlräume oder die Bindung von zwei spezifischen Bindungspartnern. Neben einer direkten Bindung an die Festphase oder das Label können die Antikörper oder Bindungspartner auch indirekt über spezifische Wechselwirkung mit anderen spezifischen Bindungspartnern an die Festphase oder das Label gebunden sein (s.a. EP-A2-0 411 945). Dies soll anhand von Beispielen näher illustriert werden: Der biotinylierte Antikörper kann über labelgebundenes Avidin an das Label gebunden werden; oder es kann ein Fluorescein-Antikörperkonjugat über festphasengebundene Anti-Fluorescein-Antikörper an die Festphase gebunden werden; oder der Antikörper kann über Immunglobulin-bindende Proteine an die Festphase oder das Label gebunden werden.

Ein weiterer Gegenstand dieser Erfindung sind erfindungsgemäße Antikörper, die als ein in vitro oder in vivo Diagnostikum oder als ein Bestandteil eines in vitro oder in vivo Diagnostikums verwendet werden.

Bei einem in vivo Diagnostikum wird der erfindungsgemäße Antikörper oder der erfindungsgemäße spezifische Bindungspartner, z.B. mit einem radioaktiven Isotop markiert, einem Lebewesen wie z.B. einem Menschen oder einem Tier appliziert. Er reichert sich bevorzugt in den Organen oder Geweben an, die pCT vermehrt enthalten oder herstellen. Durch die Detektion der Orte mit erhöhter Strahlungsintensität können beispielsweise pCT-produzierende Tumorherde lokalisiert und mit bildgebenden Verfahren räumlich dargestellt werden.

Bei einem in vitro Diagnostikum wird der nachzuweisende Analyt, z.B. Procalcitonin, in einer Probe außerhalb eines lebenden menschlichen oder tierischen Körpers nachgewiesen oder dessen Konzentration oder Menge bestimmt.

Unter einer "Probe" ist im Sinne der Erfindung das Material zu verstehen, das die nachzuweisende Substanz (Beispiele für den Begriff "Analyt" siehe EP-A2-0 515 194, Seiten 8-15) vermutlich enthält. Der Begriff Probe umfaßt beispielsweise biologische Flüssigkeiten oder Gewebe insbesondere von Menschen und Tieren wie Blut, Plasma, Serum, Sputum, Exudat, bronchoalveoläre Lavage, Lymphflüsigkeit, Synovialflüssigkeit, Samenflüssigkeit, Vaginalschleim, Feces, Urin, Liquor, Haare, Haut, Gewebeproben oder -schnitte. Ferner werden umfaßt Zellkulturproben, pflanzliche Flüssigkeiten oder Gewebe, forensische Proben, Wasser- und Abwasserproben, Nahrungsmittel, Arzneimittel. Gegebenenfalls müssen die Proben vorbehandelt werden, um den Analyten für das Nachweisverfahren zugänglich zu machen oder um störende Probenbestandteile zu entfernen. Solche Vorbehandlung von Proben mag die Abtrennung und/oder Lyse von Zellen beinhalten, das Präzipitieren, die Hydrolyse oder die Denaturierung von Probenbestandteilen wie z.B. Proteinen, die Zentrifugation von Proben, das Behandeln der Probe mit organischen Lösungsmitteln wie z.B. Alkohole, insbesondere Methanol; das Behandeln der Probe mit Detergenzien. Häufig wird die Probe in ein anderes, meistens wässriges, Medium überführtwelches mit dem Nachweisverfahren möglichst nicht interferieren soll.

Die erfindungsgemäßen Antikörper können in einem Verfahren zum quantitativen oder qualitativen Nachweis eines Analyten, Procalcitonin, in einer Probe verwendet werden.

Bei einem quantitativen Nachweis wird die Menge oder die Konzentration des Analyten in der Probe gemessen. Von dem Begriff des "quantitativen Nachweises" sind auch semiquantitative Methoden umfaßt, die nur die ungefähre Menge oder Konzentration des Analyten in der Probe erfassen oder nur zu einer relativen Mengen- oder Konzentrationsangabe dienen können. Unter einem qualitativen Nachweis ist der Nachweis des Vorhandenseins des Analyten in der Probe überhaupt oder das Anzeigen, daß die Konzentration des Analyten in der Probe unterhalb oder oberhalb eines bestimmten oder mehrerer bestimmter Schwellenwerte liegt, zu verstehen.

pCT läßt sich beispielsweise mit den erfindungsgemäßen Antikörpern immunhistochemisch in Zellausstrichen, Gewebeschnitten oder Gewebeproben nachweisen. So werden beispielsweise von dem zu untersuchenden Gewebe Gefrierschnitte oder Paraffinschnitte angefertigt. Die Schnitte werden mit den erfindungsgemäßen Antikörpern unter geeigneten Reaktionsbedingungen inkubiert. Nach Auswaschen der nichtgebundenen erfindungsgemäßen Antikörper werden die Bereiche, an die die erfindungsgemäße Antikörper gebunden haben, detektiert. Dies kann ohne weitere Zwischenschritte erfolgen, wenn die Antikörper z.B. mit Fluoreszenzlabeln versehen sind. Alternativ können die gebundenen erfindungsgemäßen Antikörper beispielsweise mit Hilfe von entsprechenden spezifischen, mit einem Label assoziierten Bindungspartnern, die an die gebundenen erfindungsgemäßen Antikörper binden können, detektiert werden.

Der Nachweis von pCT mit den erfindungsgemäßen Antikörpern kann beispielsweise auch mit Verfahren erfolgen wie beispielsweise: Western Blot, Dot Blot, Immunelektrophorese, Immunfixations-Elektrophorese, Elektroimmundiffusion, Immunpräzipitation, radiale Immundiffusion, Immunfixation, Immunchromatographie, Latex-Agglutination, turbidimetrischer oder nephelometrischer Test, homogener oder heterogener Bindungstest, Ein- oder Zweischritt-Test, Sandwich-Test, indirekter Test, kompetitiver Test, point-of-care"-Teste, etc.. Diese und andere Nachweisverfahren sind beispielsweise in "Labor und Diagnose", ed. L. Thomas, TH-Books Verlagsgesellschaft mbH, Frankfurt, 1998, Kapitel 60, oder in Laboratory Techniques in Biochemistry and Molecular Biology - An Introduction to Radioimmunoassay and Related Techniques", ed. T. Chard, Elsevier, Amsterdam, 1987, beschrieben.

Bei Bindungstesten wird der Analyt, soweit in der Probe vorhanden, an einen oder mehrere analytspezifische Bindungspartner gebunden, und es bilden sich Analyt-analytspezifische(r) Bindungspartner-Komplexe aus.

Bei homogenen Bindungstesten erfolgt keine Trennung zwischen freien und komplexgebundenen Analyten. Beispiele für homogene Immunoassays (s.a. Boguslaski & Li (1982) Applied Biochemistry and Biotechnology, 7:401-414) sind viele turbidimetrische oder nephelometrische Methoden, wobei die zum Nachweis verwendeten spezifischen Bindungspartner mit Latexpartikel assoziiert sein können; EMIT^{®}-Teste; CEDIA^{®}-Teste; Fluoreszenz-Polarisations-lmmunoassays; Luminescent Oxygen Channeling Immunoassays (EP-A2-0 515 194; Ullman et al. (1994) Proc. Natl. Acad. Sci., 91:5426-5430; Ullman et al. (1996) Clinical Chemistry ,42:1518 - 1526); etc..

Heterogene Bindungsteste sind durch einen oder mehrere Trennungsschritte und/oder Waschschritte gekennzeichnet. Die Trennung kann beispielsweise durch Immunfällung, Fällung mit Substanzen wie Polyethylenglykol oder Ammoniumsulfat, Filtration, magnetische Abtrennung, Anbindung an eine Festphase wie z.B. an ein Röhrchen, an eine Kugel, an eine Mikrotitrationsplattenvertiefung, oder an ein Filter- oder Chromatographiepapier, erfolgen. Bei heterogenen Bindungstesten ist häufig ein spezifischer Bindungspartner mit einer Komponente eines signalbildenden Systems und ein spezifischer Bindungspartner mit einer Festphase assoziiert (betreffend indirekter Bindung s.a. EP-A2-0 411 945). Es kann sich hierbei um unterschiedliche oder um die gleichen spezifischen Bindungspartner handeln, z.B. kann ein Analyt-spezifischer monoklonaler Antikörper sowohl als Fänger (z.B. als Festphasenantikörper) als auch als markierter Antikörper eingesetzt werden, wenn der Analyt mehr als ein Epitop aufweist.

Bei heterogenen Sandwich-Testen wird der Analyt üblicherweise von einem Festphasen-assoziierten spezifischen Bindungspartner und einem spezifischen Bindungspartner, der mit einer Komponente eines signalbildenden System assoziiert ist, gebunden. Bei den spezifischen Bindungspartnern kann es sich im Fall eines Sandwich-lmmunassays um Analyt-spezifische Antikörper handeln oder, wenn der Analyt selbst ein Antikörper ist, um das Antigen und/oder um ein "modifiziertes Antigen", z.B. einem gelabelten Antigen, einem Antigenteilstück, einem Antigenanalogon. Beispiele für solche Sandwichkomplexe sind: Festphase-Antikörper<>Analyt<>Antikörper-Label oder Festphase-Antigen<>Analyt(=Antikörper)<>Antigen-Label.

Eine andere Ausführungsform eines heterogenen Immunoassays ist der indirekte Immunoassay: Der Analyt ist in diesem Fall ein Antikörper. Einer der spezifischen Bindungspartner ist dessen Antigen und/oder ein modifiziertes Antigen und der andere spezifische Bindungspartner ist ein Antikörper, der den Analyten bindet, und/oder ein Immunglobulin-bindendes Protein. Beispiele für solche Komplexe, die bei einem indirekten Immunoassay gebildet werden können, sind: Festphase-Anti-IgM-Antikörper<>Analyt(=Anti-HbsAg-IgM)<>HBsAg-Label oder Festphase-HBsAg<>Analyt(=Anti-HbsAg-IgG)<>Protein A--Label.

Bei einem heterogenen kompetitiven Immunoassay konkurriert der Proben-Analyt mit einem "modifizierten Analyten", z.B. einem gelabelten Analyten, einem Analytteilstück, einem Analytanalogon, etc., um eine limitierte Anzahl Analyt-spezifischer Bindungsstellen. Beispiele zur Illustration des Prinzips: (i) Proben-Analyt konkurriert mit einem Analyt, der mit einer Komponente eines signalbildenden System assoziiert ist, um die Bindung an Festphasen-assoziierte Analyt-spezifische Antikörper oder (ii) Proben-Analyt konkurriert mit Festphasen-assoziiertem Analyt um die Bindung an Analyt-spezifische Antikörper, die mit einer Komponente eines signalbildenden System assoziiert sind.

Sandwich-Teste, kompetitive Teste und indirekte Teste können auch als homogene Testverfahren durchgeführt werden (s.a. EP-A2-0 515 194).

Der Begriff point-of-care-Teste" oder "POC-Teste" ist breit zu verstehen. Er schließt Teste ein, bei denen kein separates Analyse- oder Meßgerät zur Testdurchführung oder Testauswertung benötigt wird. POCTeste basieren in vielen Fällen auf immunchromatographische Verfahren, Immunkomplexabtrennungen per Filtration und/oder Immunfixationstechniken. Die POC-Teste sind insbesondere für Messungen vor Ort, z.B. am Krankenbett oder daheim, für den Notarzt und/oder beim niedergelassenen Arzt und weniger für das Großlabor gedacht. POC-Teste können insbesondere auch von Personen, die keine eingehende medizinischtechnische Ausbildung und Erfahrung auf dem Gebiet der Laboratoriumsmedizin haben, durchgeführt werden. Unter der Begriff "POC-Teste" sind im Sinne dieser Erfindung auch sogenannte Heimteste oder OTC-Teste zu verstehen, die von medizinischen Laien durchgeführt werden dürfen, so z.B. die diversen Schwangerschaftsteste die für den Heimgebrauch vertrieben werden. Andere POC- Teste betreffen beispielsweise den Nachweis von Herzinfarktmarkern, Drogen, Arzneimitteln, Infektions- und Entzündungsmarkern. Bei vielen POC-Testen sind oder werden im Laufe der Testdurchführung spezifische Bindungspartner an oder auf Filter- oder Chromatographiestreifen oder -scheiben assoziiert. Eine positive oder negative Nachweisreaktion kann zum Beispiel mit dem Erscheinen oder Nichterscheinen einer Farbbande in einem bestimmten Testfeld verknüpft sein und/oder dem Erscheinen oder Nichterscheinen eines bestimmten Symbols, z.B. einem "+",einem "-" und/oder der Intensität des jeweiligen Meßsignals.

Ein POC-Test für pCT kann beispielsweise so aufgebaut sein: Probe und gelabelte Antikörper, die an pCT zu binden vermögen, werden auf einen Teststreifen aufgetragen. Geeignete Label sind z.B. gefärbte Latexpartikel, kolloidales Gold, Enzyme, etc.. Sofern pCT in der Probe enthalten ist, werden sich pCT-Antikörperkomplexe ausbilden. Diese Komplexe bewegen sich mittels Kapillarkraft in Richtung auf einen Bereich, in dem Antikörper, die an andere pCT-Epitope zu binden vermögen, z.B. in Form einer Bande fixiert sind oder im Laufe des Testverfahrens fixiert werden (z.B. über eine Biotin-Avidin-Brücke). Die gelabelten pCT-Antikörperkomplexe werden in diesem Bereich gebunden und bilden mit den fixierten Antikörpern einen Sandwichkomplex aus. Die Intensität des Labelsignals ist hier proportional zur pCT-Probenkonzentration. Bei einem kompetitiven POC-Testverfahren können beispielsweise pCT und/oder pCT-Fragmente in einem Bereich des Teststreifens fixiert sein oder im Laufe des Testverfahrens fixiert werden. Dieses fixierte pCT würde mit pCT aus der Probe um die Bindung an gelabelte anti-pCT-Antikörper kompetitieren. Alternativ können auch fixierte anti-pCT-Antikörper und gelabeltes pCT für den Aufbau eines kompetitiven pCT-Testes eingesetzt werden.

Eine besonders bevorzugte Ausführungsform des erfindungsgemäßen Verfahrens ist ein nephelometrischer oder ein turbidimetrischer Test, insbesondere ein solcher Test bei dem erfindungsgemäße Antikörper - bevorzugt an Latexpartikel assoziiert - eingesetzt werden.

Ein anderer erfindungsgemäßer Gegenstand ist ein Testkit, der einen oder mehrere der erfindungsgemäßen Antikörper. In einem solchen Kit sind üblicherweise alle oder nur einige Komponenten eines Testes in abgepackter Form enthalten. Die erfindungsgemäßen Antikörper können beispielsweise mit einer oder mehreren Festphasen und/oder einer oder mehreren Komponenten eines signalbildenden Systems assoziiert sein. Der Testkit kann beispielsweise Standards; Kontrollen; sowie andere Reagenzien, wie z.B. Puffer, Waschlösungen, Meßsignal-auslösende Lösungen und/oder Enzymsubstrat; Küvetten; Pipetten und/oder Testanweisungen enthalten. Eine besonders bevorzugter erfindungsgemäßer Testkit enthält an Latexpartikel assoziierte erfindungsgemäße Antikörper.

Die erfindungsgemäßen Antikörper lassen sich auch für die Affinitätschromatographie verwenden. Unter dem Begriff "Affinitätschromatographie" ist eine Methode zur Reinigung und Isolierung von Substanzen, insbesondere Biopolymeren, zu verstehen, die auf der Tatsache beruht, daß viele Substanzen mit für sie spezifischen Bindungspartnern eine selektive, nichtkovalente, reversible Bindung eingehen können. Das Prinzip des Verfahrens besteht darin, daß der spezifische Bindungspartner an eine unlösliche Matrix (z.B. poröse Gläser, Gele auf Agarose-, Cellulose-, Dextran-, Polymer- und Kieselgelbasis) in der Regel kovalent gebunden und in Kontakt mit einer die Substanz enthaltenden Probe gebracht wird. Die gesuchte Substanz wird wegen ihrer spezifischen Wechelswirkung mit dem Matrix-gebundenen spezifischen Bindungspartner immobilisiert und zurückgehalten, während alle anderen in der Probe enthaltenen Substanzen durch Eluation abgetrennt werden. Anschließend wird das gesuchte Bioploymer mit einem geeigneten Eluationsmittel, das die nichtkovalente Bindung zwischen Substanz und spezifischen Bindungspartner aufhebt, von der Matrix abgelöst (s.a. E. Buddecke (1989) Grundrisse der Biochemie, Walter de Gruyter, Kapitel 7 "Proteine").

Ein anderer Gegenstand dieser Erfindung umfaßt erfindungsgemäße Antikörper in einem pharmazeutisch verträglichen, sterilen Injektionsmedium. Unter einem pharmazeutisch verträglichen, sterilen Injektionsmedium ist beispielsweise eine keimfreie, pyrogenfreie Lösung, z.B. Saline oder eine andere Elektrolytlösung, zu verstehen wie sie üblicherweise zur intravenösen, intramuskulären, intraperitonealen oder subkutanen Verabreichung von Arzneimitteln, Impfstoffen oder Kontrastmitteln verwendet wird.

Wieder ein anderer Gegenstand dieser Erfindung ist die Verwendung der erfindungsgemäßen Antikörper als Diagnostikum, als Bestandteil eines Diagnostikums, als Arzneimittel oder als Bestandteil eines Arzneimittels. Von der Erfindung miteingeschlossen ist auch die Verwendung der erfindungsgemäßen Antikörper und/oder der erfindungsgemäßen spezifischen Bindungspartner zur Behandlung der SIRS ("systemic inflammatory response syndrome"), der Sepsis und/oder Tumore, insbesondere bösartige pCT-produzierende Tumore. SIRS ist eine systemische, entzündliche Antwort auf eine Gewebeschädigung mit den Zeichen einer entfernt ablaufenden Organdysfunktion. Ferner wird von der Erfindung umfaßt ein Verfahren zur Herstellung eines Arzneimittels, beispielsweise zur Behandlung von Tumoren, der Sepsis und/oder der SIRS, enthaltend die erfindungsgemäßen Antikörper.

Die erfindungsgemäßen Antikörper können zur Behandlung intrakorporal oder auch extrakorporal eingesetzt werden. Die erfindungsgemäßen Antikörper können durch radioaktive Isotope und/oder durch die Anbindung pharmakologisch wirksamer Substanzen in ihrer Wirksamkeit verstärkt werden. Die therapeutische Applikation von anti-pCT-Antikörpern ist in WO 98/33524 näher erläutert. Darüber hinaus könnte pCT aus dem Blut eines Patienten beispielsweise auch mit einem Dialyse-analogen Verfahren entfernt werden, wobei das Blut oder das Plasma extrakorporal mit sterilen und fixierten erfindungsgemäßen Antikörpern in Kontakt gebracht wird.

Ein anderer Gegenstand dieser Erfindung ist ein Verfahren zur Herstellung eines erfindungsgemäßen Antikörpers, welches dadurch gekennzeichnet ist, daß zur Immunisierung ein Peptid verwendet wird mit der Aminosäuresequenz Val-Gly-Ala-Pro-Gly-Lys-Lys-Arg-Asp-Met-Ser-Ser.

Der Begriff "Peptide" umfaßt Säureamide, die bei Hydrolyse in Aminosäuren zerfallen, beispielsweise Aminosäurepolymere wie z.B. Polypeptide, Oligopeptide, Proteine oder Proteinfragmente. Sind nicht mehr als zehn Aminosäuren miteinander verknüpft, so spricht man in der Regel von Oligopeptiden, darüber hinaus von Polypeptiden. Oligopeptid gemäß Definition dieser Erfindung umfaßt auch noch Aminosäureketten von bis zu etwa 20 Aminosäuren.

Ein Gegenstand dieser Erfindung ist auch das Oligopeptid mit der Aminosäuresequenz Val-Gly-Ala-Pro-Gly-Lys-Lys-Arg-Asp-Met-Ser-Ser. Dieses Peptid kann als Immunisierungsantigen zur Herstellung der erfindungsgemäßen Antikörper verwendet werden.

Das als Immunisierungsantigen verwendete Peptid kann ungebunden und/oder trägerbunden zur Immunisierung verwendet werden. Typische Träger sind beispielsweise Proteine, wie z.B. Ovalbumin, Albumin oder Hämocyanin, oder Polymere, wie z.B. Polyethylenglykol, Polyacrylamid oder Poly-d-Glutamin-d-Lysin. Die Peptide können beispielsweise mit Hilfe von Carbodiimid oder Glutaraldehyd an diese Träger gebunden werden oder auch mittels eines bifunktionalen Reagenzes, welches auch als Abstandhalter ("Spacer") wirken kann (Beispiele und Kopplungsmethoden s. z.B. Wong S. (1993) Chemistry of Protein Conjugation and Cross-Linking, CRC Press, Inc, Boca Raton).

Das Immunisierungsantigen kann beispielsweise in Phosphat-gepufferter Saline aufgenommen werden und mit Freund'schem Adjuvans versetzt werden. Diese Emulsion kann dann z.B. intradermal, intraperitoneal und/oder subkutan einem Tier, beispielsweise einem Kaninchen, einer Maus, einer Ratte, einem Meerschweichen, einem Pferd, einem Schaf, einer Ziege, einem Huhn, etc., appliziert werden. Booster-Injektionen, wobei das Immunisierungsantigen auch mit inkomplettem Freund'schen Adjuvans emulgiert sein kann, können helfen, die Immunantwort zu steigern.

Je nach gewünschtem Verwendungszweck ist es vorteilhaft nur Teile der Antikörper, wie z.B. Fab-, F(ab')₂₋, oder Fab'-Fragmente, einzusetzen. Diese können beispielsweise mit den dem Fachmann bekannten enzymatischen Spaltverfahren (s.a. z.B. Harlow & Lane (1988) Antibodies: A Laboratory Manual, Cold Spring Harbor Laboratory, Cold Spring Harbor) erzeugt werden.

Die Antigenbindungsstellen eines Antikörpers befinden sich in den sogenannten variablen Domänen, die durch die V-Gene kodiert sind. Mit den bekannten gentechnischen Methoden (s. z.B. Sambrook et al. (1989) Molecular Cloning: A Laboratory Manual, Cold Spring Harbor Laboratory, Cold Spring Harbor, 2nd edition; McCafferty et al. (1990) Nature 348:552-554) kann auch die entsprechende Nukleinsäuresequenz eines erfindungsgemäßen Antikörpers ermittelt werden sowie dadurch auch die entsprechende Aminosäuresequenz, sofern diese noch nicht per Aminosäuresequenzierung bereits bekannt war. Als Ausgangsmaterial für derartige Analysen können die Hybridomazellen bzw. die Antikörper produzierenden Immunzellen immunisierter Tiere eingesetzt werden.

In Kenntnis der Nuklein- und/oder Aminosäuresequenz können mit Hilfe üblicher gentechnologischer und molekularbiologischer Methoden (s.a. Johnson & Chiswell (1993) Current Opinion in Structural Biology, 3:564-571) dann humanisierte, chimäre, bi-oder oligospezifische Antikörper sowie von der "complementarity determining region" abgeleitete Peptide ("minimal recognition units"), single-chain Fragmente, und/oder funktionelle Fusionsprodukte, z.B. rekombinant hergestellte Antikörper-Enzym-Konstrukte, hergestellt werden (s. z.B. Larrick & Fry (1991) Human Antibodies and Hybridomas, 2:172-189; Kitano et al (1986) Appl. Microbiol. Biotechnol, 24:282-286; Thompson et al. (1986) J. Immunol. Methods, 94 7-12), die an Procalcitonin nicht jedoch an freies Calcitonin, freies Katacalcin und freies N-Procalcitonin binden. Mit solchen unter dem Begriff "Antikörper" eingeschlossenen Peptiden kann beispielsweise eine Verringerung der Immunogenität und/oder eine verstärkte Wirksamkeit bei Verabreichnung als Arzneimittel oder in vivo Diagnostikum erzielt werden und/oder es ergeben sich Vorteile für den Einsatz als oder in einem in vitro Diagnostikum. Die Antikörper sind auch herstellbar ggf. unter Zuhilfenahme gentechnologischer Methoden in pflanzlichen - wie z.B. Hefezellen - (Fischer et al. (1999) Biol. Chem., 380:825-839; Hiatt et al. (1992) Genetic Engineering, 14:49-64)), tierischen und prokaryontischen Zellen (s. z.B. WO 95/25172) sowie isolierten menschlichen Zellen.

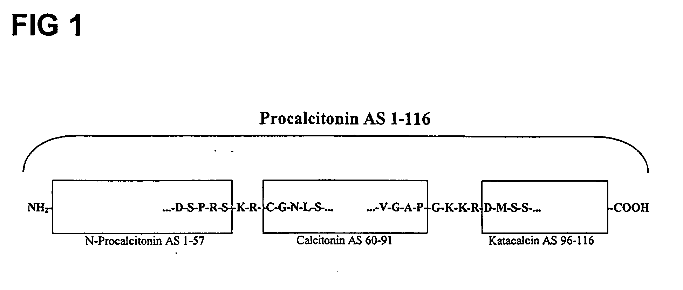

Ein weiterer Gegenstand dieser Erfindung ist die Hybridomazelllinie 98-31/04. Diese Hybridomazelllinie wurde am 16.12.1999 bei der DSMZ Deutsche Sammlung von Mikroorganismen und Zellkulturen GmbH, Mascheroder Weg 1b, Braunschweig, Deutschland, mit der Eingangsnummer DSM ACC2437 hinterlegt
FIG 1 stellt eine schematische Darstellung von humanem pCT und dessen proteolytische Spaltprodukte dar; AS = Aminosäure(n).

### Beispiele:

### Beispiel 1: Peptidsynthese:

Zwei Peptide "P1" und "P2" entsprechend den Aminosäuren 53 - 64 und 88 - 99 des humanen Procalcitonins (s.a. Fig 1) mit der Sequenz
P1: Asp-Ser-Pro-Arg-Ser-Lys-Arg-Cys-Gly-Asn-Leu-Ser
P2: Val-Gly-Ala-Pro-Gly-Lys-Lys-Arg-Asp-Met-Ser-Ser
wurden wie folgt synthetisiert:

(P1 und P2 werden an einem Applied Biosystem, Model 431A Peptidsynthesizer mit 9-Fluroenylmethyloxicarbonyl (Fmoc) Aminosäurenchemie synthetisiert. Die vorhandenen Schutzgruppen wurden durch Behandlung mit Trifluoressigsäure abgespalten .

### Beispiel 2: Kopplung der Peptide an einen Träger

Die Peptide wurden an Rinderserum-Albumin (RSA, Centeon Pharma, Marburg, Germany) gekoppelt (s.a. Rusin et al. (1992) Biosens. Bioelectron., 7:367-373; Kitagawa et al. (1983) J. Biochem., 94:1165-1172).

Im einzelnen: 100 mg RSA werden in 20 ml 0,1M Lithium-Borat-Puffer pH 8,0 mit 41,8 mg N-gamma-Maleimidobytyryloxysuccinimide (GMBS) (Calbiochem-Novabiochem GmbH, Bad Soden, Deutschland) versetzt und 30 Minuten bei 20°C inkubiert. Anschließend wird das Reaktionsgemisch gegen 0,1 M NaH₂(PO₄) pH 6,0 umgepuffert (RSA-GMBS-Lösung).
17,5 mg P1 oder P2 werden in 1,75 ml 0,1M Lithium-Borat-puffer pH 8,0 gelöst, und 2,1 mg S-acetylmercaptobernsteinsäure-anhydrid (Fluka Chemie GmbH, Deisenhofen, Deutschland) gelöst in Dioxan (22,2mg/ml) zugegeben und 30 Minuten bei 20°C inkubiert. Anschließend werden 475 µl 1M Hydroxylamin-Lösung zugesetzt und weitere 15 Minuten bei 20°C inkubiert. Das erhaltene Reaktionsgemisch wird mit 16 ml RSA-GMBS-Lösung versetzt und 2 Stunden bei 20°C inkubiert. Anschließend wird die Reaktion durch Zugabe von 0,1 M N-ethylmalemid-Lösung abgestoppt und gegen Phosphat-gepufferter Saline pH 7,2 umgepuffert.

### Beispiel 3: Herstellung von monoklonalen Antikörpern

### a) Immunisierung von Mäusen

BALB/c Mäuse wurden jeweils mit 20 µg P1-RSA-Konjugat bzw 20 µg P2-RSA-Konjugat in kompletten Freund'schen Adjuvans intraperitoneal immunisiert. Nach 4 Wochen erfolgte ein Booster mit jeweils 20µg P1-RSA-Konjugat bzw 20 µg P2-RSA-Konjugat in inkompletten Freund'schen Adjuvans ( Fa. ICN Biomedical GmbH, Eschwege, Deutschland) und nach 8 Wochen mit jeweils 20 µg P1-RSA-Konjugat bzw. 20 µg P2-RSA-Konjugat ohne Freund'schen Adjuvans. Die letzten 3 Tage vor der Fusion wurden die Mäuse intravenös mit jeweils 20 µg P1-RSA-Konjugat bzw. 20 µg P2-RSA-Konjugat geboostert.

### b) Fusion

Nach dem Töten der Mäuse durch CO₂-Inhalation wurden die Milzen entnommen und Einzelzellsuspensionen in serumfreien Dulbeccos modifiziertem Eagle Medium (DMEM, Fa. CC Pro GmbH, Neustadt/W, Deutschland) hergestellt. Die Zellen wurden zentrifugiert (652 g) und 2 x in DMEM gewaschen. Anschließend wurde die Zellzahl mittels Trypanblau-Färbung bestimmt. Zu etwa 10⁸ Milzzellen wurden 2×10⁷ Myelomzellen (Sp2/0) gegeben. Nach dem Zentrifugieren (360 g) wurde der Überstand verworfen, 1ml Polyethylenglycol-Lösung (PEG 4000, Fa. Merck Eurolab, Bruchsal, Deutschland; ca. 50%ig in DMEM) auf das Zellpellet gegeben und nach Resuspension 1 Minute bei 37°C inkubiert. Anschließend wurde tropfenweise ca. 10 ml DMEM zugegeben und 2 bis 4 Minuten bei Raumtemperatur inkubiert. Die fusionierten Zellen wurden abzentrifugiert (326 g) und das Pellet in DMEM + 20% FKS (fötales Kälberserum, Fa. Bio Witthaker Europe, Verviers, Belgien) + HAT-Lösung (Fa. CC Pro GmbH, Neustadt/W, Deutschland) resuspendiert und in 24 Well-Zellkulturplatten (Fa. Costar) abgefüllt. Die ungefähre Zellkonzentration pro Well betrug 5×10⁴ bis 5×10⁶ Zellen.

2 - 3 Wochen später wurden die entstandenen Zellkolonien (Hybride) entnommen und in neue Kulturplatten überführt.

### c) Bestimmung der Antikörperspezifität

Die Spezifität der in die Zellkultur abgegebenen Antikörper wurden in einem ersten Testschritt mit Hilfe von Immunisierungsantigen-beschichteten Mikrotiterplatten (Fa. Nunc, Typ B), Beschichtung 0,2 µg/ml ≈ 0,003 µg/Vertiefung, getestet.

In jede Vertiefung der Mikrotitterplatte wurden 100 µl Zellkulturüberstand (Verdünnung 1:2) pipettiert und 1 Stunde bei +15 bis +25°C inkubiert. Nach zweimaligen Waschen der Platte mit Waschlösung-POD (OSEW; Fa. Dade Behring, Marburg, Deutschland) wurden in jede Vertiefung 100 µl anti-Maus IgG/F(ab')₂-POD-Konjugat (Fa. Dade Behring, Marburg, Deutschland) eingefüllt und 1 Stunde bei +15 bis +25°C inkubiert. Nach weiterem zweimaligen Waschen der Platte wurde in jede Vertiefung 100 µl Chromogen TMB-Lösung (Fa. Dade Behring, Marburg, Deutschland) eingefüllt und weitere 30 Minuten bei +15 bis +25°C inkubiert. Nach der Inkubation wurde in jede Vertiefung 100 µl Stopplösung POD (Fa. Dade Behring, Marburg. Deutschland) eingefüllt und die Mikrotitterplatte am BEP II (Behring-ELISA-Prozessor II, Fa. Dade Behring, Marburg, Deutschland ) bei 450 nm ausgewertet.

In einem 2. Testschritt wurden die Hybride wie oben beschrieben überprüft mit Hilfe von Mikrotiterplatten (Fa. Nunc, Typ B), die mit folgenden Peptiden beschichtet waren:
i. Rekombinantes humanes pCT (0,03 µg/Vertiefung). Die Herstellung von rekombinanten pCT ist im Detail in der am 22. Dezember 1999 beim Deutschen Patent- und Markenamt parallel eingereichten Anmeldung mit dem Titel "Humanes Procalcitonin, dessen Herstellung und Verwendung" beschrieben (Aktenzeichen 199 62 434.8). Ein zur Expression von pCT in E.coli geeignetes Plasmid wurde am 16.12.1999 bei der DSMZ Deutsche Sammlung von Mikroorganismen und Zellkulturen GmbH unter der Nummer DSM 13203 hinterlegt. Dieses Plasmid kann mittels Standardmethoden (Current Protocols in Molecular Biology, Wiley, 1997) in einen geeigneten E. coli Stamm (z.B. JM109; Stratagene, LaJolla, USA) transformiert werden. Klone können nach Ausplattieren des Transformationsansatzes auf LB-Agarplatten welche 50 µg/ml Ampicillin als Selektionsmedium enthalten gewonnen werden. Die Expression des Procalcitonins kann nach folgendem Prozedere erfolgen: Frisch mit dem Expressionsplasmid transformierte JM109 werden über Nacht bei 37°C unter Schütteln in LB-Medium mit 100 µg/ml Ampicillin angezogen und dann 1:50 in 1 I frischem LB-Medium (Ampicillin 100µg/ml) verdünnt und weiter bei 37°C geschüttelt, und bei einer OD₆₀₀ von 0,4 mit 2 mM IPTG für 3h induziert. Bei Einhaltung dieser optimierten Bedingungen wurden reproduzierbar ca. 13 mg Fusionsprotein aus 11 Kultur nach einer Reinigung unter nativen Bedingungen über Metallaffinitätschromatographie nach Angaben des Herstellers (Talon Metal Affinity Resin, Clontech, Palo Alto, USA) und anschließender Gelfiltration über Superdex 75 HiLoad (Amersham Pharmacia), erhalten.
ii. Calcitonin human RSA-Konjugat (0,5 µg/Vertiefung, Fa, Bachem, Prod. Nr.: H-2250)
iii. Katacalcin human (PDN-21) RSA-Konjugat ( 0,5µg/Vertiefung, Fa, Peninsula, Prod. Nr.: 6004)
iv. Calcitonin N-Terminal Flanking Peptide RSA-Konjugat(0,5 µg/Vertiefung, Fa, Bachem, Prod. Nr.: H-3076) = humanes N-Procalcitonin

Die Ergebnisse sind in Tabelle 1 aufgelistet.

**Tabelle 1: Bestimmung der Antikörperspezifität durch Auswertung der Mikrotiterplatten am BEP II (Behring-ELISA-Prozessor II) bei 450 nm.**

| | Extinktion bei 450 nm | | | | | |
|---|---|---|---|---|---|---|
| Hybrid/Klon | Peptid 1 | Peptid 2 | Rekombinan tes humanes Procalcitonin | Calcitonin | Katacalcin | N-Procalcitonin |
| 98-47/44 | 0,975 | negativ | 0,100 | 0,561 | 2,5 | Negativ |
| 98-31/04 | negativ | 1,715 | 0,290 | negativ | negativ | Negativ |
| 98-31/74 | negativ | 2,374 | negativ | 0,118 | 0,149 | Negativ |

| | | | | | | |
|---|---|---|---|---|---|---|
| negativ = Extinktion 450 nm < 0,1 bzw. bei Verdünnung des untersuchten Hybride keine Abstufung des Signals | | | | | | |

### d) Klonierung

Einzelne Zellen von Hybriden, die die erfindungsgemäßen Antikörper produzieren (Bindung an humanes pCT nicht jedoch an freies Calcitonin, freies Katacalcin und freies N-Procalcitonin), wurden mit einem Mikromanipulator (Fa. Leitz, Wetzlar, Deutschland) kloniert. Der so erhaltene Klon 98-31/04 wurde am 16.12.1999 bei der DSMZ Deutsche Sammlung von Mikroorganismen und Zellkulturen GmbH, Mascheroder Weg 1b, Braunschweig, Deutschland, unter der Eingangsnummer DSM ACC2437 hinterlegt.

### e) Bestimmung der Antikörpersubklasse

Die Subklasse des Antikörpers 98-31/04 wurde mittels IsoStrip™-Mouse Monoclonal Antibody Isotyping Kit- der Fa. Boehringer Mannheim, Deutschland als IgG₁ bestimmt.

### f) Produktion der Antikörper

Für die Produktion größerer Mengen Antikörper werden die entsprechenden Zellklone in Rollerflaschen (Fa. Corning Costar Deutschland, Bodenheim) überführt, und bis zum gewünschten Endvolumen bei +37°C expandiert. Danach wird die Rollerkultur-Suspension zur Entfernung der Zellen über 0,22 µm filtriert. Die jetzt zellfreie Antikörperlösung wird über Ultrafilter (Trenngrenze 30 000 Dalton) ankonzentriert und anschließend aufgereinigt.

### g) Reinigung der Antikörper

Die erhaltene Antikörperlösung wird gegen 0,14 M Phosphatpuffer pH 8,6 umgepuffert und auf ein mit rProtein A Sepharose Fast Flow (Fa. Amersham Pharmacia) gefüllte Chromatographiesäule aufgetragen (pro 10 mg zu reinigender Antikörper werden 1 ml rProtein A Sepharose Fast Flow eingesetzt). Alle nicht gebundenen Komponenten werden durch Waschen der Säule mit 0,14 M Phosphatpuffer pH 8,6 entfernt. Der gebundene Antikörper wird mit 0,1 M Zitronensäure pH 3,0 von der Säule eluiert und gegen 0,05 M Natriumacetat + 0,5 M NaCl + 0,05 M Tris + 0,01% Natriumazid pH 7,0 dialysiert.

### 4. Beispiel: Nachweis von pCT in einer Probe

### a) Bindung von MAK an Latexpartikel

An Latexpartikel hergestellt nach EP-0246 446 mit einem Durchmesser von 250 bis 310 nm wurde jeweils ein erfindungsgemäßer monoklonaler Antikörper und ein gegen Katacalcin gerichteter monoklonaler Antikörper gebunden:

Das verwendete Latexpolymerisat wurde mit dest. Wasser auf einen Feststoffgehalt von 4 Gew. % verdünnt. Die zu bindenden Antikörper wurden mit 0,05 M Natriumacetat + 0,5 M NaCI + 0,05 M Tris + 0,01% Natriumazid pH 7,0 auf einen Proteingehalt von 5 mg/ml verdünnt. 1 ml des obengenannten Polymerisats wurde mit 200 µl Antikörper-Lösung gemischt. Dann wurden 0,050 ml einer 20 %igen Lösung von Tween 20 (Fa. Merck Eurolab, Darmstadt, Deutschland) hinzugegeben und das Ganze nochmals gemischt. Hierzu wurde 0,025 ml 1 N HCl zugegeben, so daß ein pH-Wert von ca. 3 erreicht wurde. Nach einer Inkubationszeit von 30 min. bei Raumtemperatur wurde 0,25 ml 1M Phosphatpuffer pH 6,5 und 0,25 ml Natriumcyanborhydrid-Lösung (25 mg/ml) hinzugesetzt und gut durchgemischt. Anschließend erfolgte eine Inkubation von einer Stunde bei Raumtemperatur.

Dieser Beladungsansatz wurde sodann 30 min. bei ca. 50 000 g zentrifugiert. Der Überstand wurde verworfen. Der Rückstand wurde in 4 ml Imidazolpuffer pH 8,1 (5g/L Imidazol, 40g/L Saccharose, 1g/L Humanalbumin) resuspendiert. Anschließend erfolgte eine Ultraschall-Behandlung (Bronson Sonyfier B15) für 30 Sekunden. Das so redispergierte Reagenz wurde im Volumenverhältnis 1:7,5 mit dem zuvor genannten Imidazolpuffer verdünnt und nochmals 30 Sekunden mit Ultraschall behandelt.

### b) Nachweis von pCT

Die nach Beispiel 4a) durch Bindung des erfindungsgemäßen Antikörpers und des Antikörpers gegen Katacalcin an Latexpartikel hergestellten Reagenzien wurden in einem Volumenverhältnis 1+1 gemischt und zur Messung von pCT in Patientenseren am Behring Nephelometer Analyser (BNA, Dade Behring, Marburg, Deutschland) eingesetzt. Das gemischte Reagenz wird bei Mischung mit pCT- haltigen Proben agglutiniert. Die Intensität des Streulichts im BNA ist von der pCT-Konzentration der Probe abhängig. Zur Messung wurden 100 µl Probe (1 Normalserum , 3 pCT-haltige Patientenproben (BRAHMS LUMItest^{®} PCT, > 100 ng/ml pCT)) mit 100 µl N-Diluens (Dade Behring, Marburg, Deutschland) und mit 40 µl des gemischten Reagenzes in der Reaktionskuvette gemischt und nach 12 min. die Veränderung des Meßsignals (in Bit) am BNA gemessen. Die Ergebnisse sind in Tabelle 2 zusammengefasst.

**Tabelle 2**

| **Probe** | **Meßsignal BNA in Bit** |
|---|---|
| Normalserum | -58 |
| pCT-haltiges Serum 1 | 212 |
| pCT-haltiges Serum 2 | 197 |
| pCT-haltiges Serum 3 | 592 |

## Patentansprüche

1. Antikörper, der an ein Peptid mit der Aminosäuresequenz Val-Gly-Ala-Pro-Gly-Lys-Lys-Arg-Asp-Met-Ser-Ser bindet, **dadurch gekennzeichnet, daß** er Procalcitonin, nicht jedoch freies Calcitonin, freies Katacalcin und freies N-Procalcitonin bindet und daß er von der Hybridomazellinie 98-31/04 (DSM ACC2437) produziert wird.

2. Antikörper nach Anspruch 1, **dadurch gekennzeichnet, daß** der Antikörper mit einer Festphase und/oder einer Komponente eines signalbildenden Systems assoziiert ist.

3. Antikörper nach Anspruch 1 zur Verwendung als in vitro oder in vivo Diagnostikum oder als Bestandteil eines in vitro oder in vivo Diagnostikums.

4. Antikörper nach Anspruch 1 zur Verwendung in einem Verfahren zum quantitativen oder qualitativen Nachweis von Procalcitonin, in einer Probe.

5. Antikörper nach Anspruch 1 zur Verwendung in der Affinitätschromatographie.

6. Testkit enthaltend den Antikörper nach Anspruch 1.

7. Verfahren zur Herstellung des Antikörpers nach Anspruch 1, **dadurch gekennzeichnet, daß** zur Immunisierung das Peptid mit der Aminosäuresequenz Val-Gly-Ala-Pro-Gly-Lys-Lys-Arg-Asp-Met-Ser-Ser verwendet wird und daß derjenige Antikörper ausgewählt wird, der Procalcitonin, nicht jedoch freies Calcitonin, freies Katacalcin und freies N-Procalcitonin bindet.

8. Verfahren nach Anspruch 7, **dadurch gekennzeichnet, daß** das zur Immunisierung verwendete Peptid trägergebunden vorliegt.

9. Hybridomazellinie mit der Bezeichnung 98-31/04, die bei der DSMZ unter der Eingangsnummer DSM ACC2437 hinterlegt wurde.

10. Peptid mit der Aminosäuresequenz Val-Gly-Ala-Pro-Gly-Lys-Lys-Arg-Asp-Met-Ser-Ser.

## Claims

1. An antibody which binds to a peptide having the amino acid sequence Val-Gly-Ala-Pro-Gly-Lys-Lys-Arg-Asp-Met-Ser-Ser and which binds procalcitonin but not free calcitonin, free katacalcin and free N-procalcitonin and which is produced by the hybridoma cell line 98-31/04 (DSM ACC2437).

2. An antibody as claimed in claim 1 which is associated with a solid phase and/or a reporter system component.

3. An antibody as claimed in claim 1 which is used as an in vitro or in vivo diagnostic agent or as an ingredient of an in vitro or in vivo diagnostic agent.

4. An antibody as claimed in claim 1 which is used in a method for quantitative or qualitative detection of procalcitonin in a sample.

5. An antibody as claimed in claim 1 which is used in affinity chromatography.

6. A test kit comprising the antibody as claimed in claim 1.

7. A process for preparing the antibody as claimed in claim 1, which comprises using for the immunization the peptide having the amino acid sequence Val-Gly-Ala-Pro-Gly-Lys-Lys-Arg-Asp-Met-Ser-Ser and selcting that antibody which binds procalcitonin but not free calcitonin, free katacalcin and free N-procalcitonin.

8. The process as claimed in claim 7 wherein the peptide used for the immunization is present in carrier-bound form.

9. A hybridoma cell line which is named 98-31/04 and which was deposited with the DSMZ under accession number DSM ACC2437.

10. A peptide having the amino acid sequence Val-Gly-Ala-Pro-Gly-Lys-Lys-Arg-Asp-Met-Ser-Ser.

## Revendications

1. Anticorps qui se lie à un peptide ayant la séquence d'acides aminés Val-Gly-Ala-Pro-Gly-Lys-Lys-Arg-Asp-Met-Ser-Ser, **caractérisé en ce qu'**il se lie à la procalcitonine, mais pas à la calcitonine libre, à la katacalcine libre ni à la N-procalcitonine libre et **en ce qu'**il est produit par la lignée cellulaire d'hybridome 98-31/04 (DSM ACC2437).

2. Anticorps selon la revendication 1, **caractérisé en ce que** l'anticorps est associé à une phase solide et/ou à un composant d'un système générateur de signal.

3. Anticorps selon la revendication 1, pour utilisation en tant qu'agent de diagnostic in vitro ou in vivo ou en tant que composant d'un agent de diagnostic in vitro ou in vivo.

4. Anticorps selon la revendication 1, pour utilisation dans un procédé pour la détection quantitative ou qualitative de procalcitonine dans un échantillon.

5. Anticorps selon la revendication 1, pour utilisation en chromatographie d'affinité.

6. Kit contenant l'anticorps selon la revendication 1.

7. Procédé pour la production de l'anticorps selon la revendication 1, **caractérisé en ce qu'**on utilise pour l'immunisation le peptide ayant la séquence d'acides aminés Val-Gly-Ala-Pro-Gly-Lys-Lys-Arg-Asp-Met-Ser-Ser et **en ce qu'**on choisit ledit anticorps qui se lie à la procalcitonine, mais pas à la calcitonine libre, à la katacalcine libre ni à la N-procalcitonine libre.

8. Procédé selon la revendication 7, **caractérisé en ce que** le peptide utilisé pour l'immunisation est présent fixé sur un support.

9. Lignée cellulaire d'hybridome portant la désignation 98-31/04, qui a été déposée à la DSMZ (Deutsche Sammlung für Mikroorganismen und Zellkulturen) sous le numéro de dépôt DSM ACC2137.

10. Peptide ayant la séquence d'acides aminés Val-Gly-Ala-Pro-Gly-Lys-Lys-Arg-Asp-Met-Ser-Ser.


### SEQUENZPROTOKOLL

<110> Dade Behring Marburg GmbH
<120> Gegen Procalcitonin gerichtete Antikörper, ihre Herstellung und Verwendung
<130> MA1237
<140>
   <141>
<150> 19962417.8
   <151> 1999-12-22
<150> 10016277.0
   <151> 2000-04-03
<150> 10041215.7
   <151> 2000-08-22
<160> 4
<170> PatentIn Ver. 2.1
<210> 1
   <211> 6
   <212> PRT
   <213> Homo sapiens
<400> 1
   Arg Ser Lys Arg Cys Gly
    1               5
<210> 2
   <211> 12
   <212> PRT
   <213> Homo sapiens
<400> 2
   Asp Ser Pro Arg Ser Lys Arg Cys Gly Asn Leu Ser
    1               5                  10
<210> 3
   <211> 6
   <212> PRT
   <213> Homo sapiens
<400> 3
   Pro Gly Lys Lys Arg Asp
    1               5
<210> 4
   <211> 12
   <212> PRT
   <213> Homo sapiens
<400> 4
   Val Gly Ala Pro Gly Lys Lys Arg Asp Met Ser Ser
     1               5                   10